# NEW EUROPEAN PATENT SPECIFICATION

(11) **EP 0 707 068 B2**
(45) Date of publication and mention of the opposition decision: **11.10.2006**
(45) Mention of the grant of the patent: 31.10.2001
(21) Application number: 95114460.9
(22) Date of filing: 22.05.1985
(51) Int. Cl.: C12N 15/81

(54) **Yeast vector**
Hefevektor
Vecteur de levure

(30) Priority: 22.05.1984 US 612796
(43) Date of publication of application: 17.04.1996
(62) Divisional of application: 85303625.9
(73) Proprietor: ROBERT ROGERS YOCUM, Lexington, Massachusetts 02420-2600 (US)
(72) Inventor: Yocum, Robert Rogers, Lexington,Massachusetts 02320-2600 (US)
(74) Representative: Wallace, Sheila Jane

(56) References cited:
- EP-A- 0 068 740
- EP-A- 0 096 430
- AMERICAN JOURNAL 0F TROPICAL MEDICINE AND HYGIENE, vol. 29, no. 5 (supplement), 1980 pages 1089-1092, J.DAVIES AND A.JIMENEZ 'A new selective agent for eukaryotic cloning vectors'
- GENE, vol. 26, no. 2/3, December 1983 AMSTERDAM NL, pages 243-252, T.D.WEBSTER AND R.C.DICKSON 'Direct selection of Saccharomyces cerevisiae resistant to the antibiotic G418 following transformation with a DNA vector carrying the kanamycin-resistance gene of Tn903'

## Description

This invention relates to genetic engineering in yeast.

Technology currently exists for introducing heterologous (i.e., modified or foreign) genes into laboratory strains of yeast of the genus Saccharomyces, particularly S. cerevisiae. Two types of plasmid vectors have been used for this purpose, replicating and integrating. Replicating vectors contain an origin of DNA replication that functions in yeast, so that the plasmid is maintained extrachromosomally, as a circular episome. Integrating vectors do not necessarily contain such an origin and may be stably maintained by insertion into a yeast chromosome.

Both types of plasmids can be introduced into yeast cells by standard transformation methods. Since successful, uptake and establishment of plasmid DNA by competent yeast cells is a relatively rare event (<10⁻³), a selection mechanism is required to allow identification of transformants.

Most commonly, selection is accomplished by introducing auxotrophic mutations into the recipient yeast strain. The commonly used mutations are ura3, leu2, trpl, and his3. The plasmid of interest bears a wild type copy of one of these genes. Since the wild type copy on the plasmid is dominant to the host chromosomal allele, selection for cells that receive the plasmid is easily accomplished on a minimal medium lacking the nutrient that is required by the auxotrophic host cell.

There have also been reports of the use of antibiotic resistance to select transformed cells. Replicating vectors have been described that are based on the sensitivity of most Saccharomyces strains to the commercially available neomycin analog, antibiotic G418 (also known as "Geneticin"^{™}); Jimenez et al. (1980) Nature 287, 868; Hollenberg (1982) in Current Topics in Microbiology and Immunology, Höfschneider et al., eds. (Springer-Verlag, NY); Webster et al. (1983) Gene 26, 243. Webster et al. also describe an integrating plasmid vector which had not been directly selected for by resistance to G418. The vectors described in the above references contain a gene, called kan^{r}, neo^{r}, or G418^{r} from the bacterial transposon Tn903, and the examples of replicating vectors contain a yeast origin of replication; in all of these examples, both replicating and integrating, the bacterial gene is preceded by its native bacterial promoter.

Another replicating vector has been described which contains the gene for resistance to the antibiotic hygromycin B under the control of a yeast promoter; Gritz et al. (1983) Gene 25, 178.

EP-A-0068740 relates to a recombinant DNA cloning vector comprising (a) a eukaryotic promoter, (b) one or two different structural genes and associated control sequence that convey resistance to either or both antibiotics hygromycin and G418 when transformed into a host cell that is sensitive to either or both antibiotics for which resistance is conveyed, said host cell being susceptible to transformation, cell division, and culture, and (c) a prokaryotic, replicon, said replicon being functional when said host cell is prokaryotic, subject to the limitations that the one or two structural genes and associated control sequence are adjacent to and, in a eukaryotic host cell, transcribed from the eukaryotic promoter, that a signal gene and associated control sequence conveys resistance to either but not both hygromycin B and G418, and that the gene, conveying resistance to G418 does not code for the enzyme phosphotransferase. However, this document does not enable a skilled person to construct a DNA sequence in which G418 resistance is functionally coupled to a yeast promoter sequence.

According to the present invention there is provided a vector containing a DNA sequence capable of integration into a yeast chromosome comprising a yeast promoter sequence functionally coupled to a gene coding for resistance to antibiotic G418, such that the DNA sequence is capable of being directly selected for in the integrated state in a yeast cell transformed with said DNA.

The vector may, for example, be as described in any of claims 2 to 5. The vector may be present in a host yeast call. It may include a gene heterologous to the host yeast cell (i.e., a non-yeast gene, a modified gene, a gene from a different yeast strain, or a homologous gene from a different chromosomal location). The vector can be used to transform the host cells; transformants are selected on the basis of antibiotic resistance.

In preferred embodiments, the vector also includes a sequence which is homologous with a sequence (a "target" sequence) of a host chromosome, to facilitate integration. Preferably, the homologous sequence is separate from the control sequence which controls the antibiotic resistance gene, and preferably the target is a region where the metabolism of the host cell will not be interfered with.

In other preferred embodiments, the heterologous gene encodes an enzyme, e.g., glucoamylase (which enables the generation of glucose from starch by the yeast cell), and the host cell participates in a process, e.g., the production of dough, which employs a product of the metabolism of the cell e.g. carbon dioxide.

In other preferred embodiments, the antibiotic resistance gene and the heterologous gene are under the control of different promoters, the promoter controlling the heterologous gene preferably being the more highly expressed of the two. The preferred integration method is one which results in the depositing of the heterologous gene in the host yeast cell chromosome, to the exclusion of much of the remainder of the vector DNA, providing greatly increased stability. Exclusion of this DNA also eliminates a potential source of interference with a characteristic, e.g., flavour, of the end product.

The vector may comprise a gene encoding glucoamylase, said vector enabling the expression of said glucoamylase in a host yeast cell.

The vector may comprise a gene encoding malolactic enzyme or malate permease, said vector enabling the expression of said malolactic enzyme or malate permease in a host yeast cell.

The vector may include a sequence homologous with a sequence of a chromosome of a host yeast cell, wherein integration of said vector in said sequence of said chromosome does not interfere with the metabolism of said host yeast cell.

Integrating vectors in accordance with this invention provide stability over generations of host divisions in the absence of selection, an important advantage in industrial fermentation processes; replicating vectors can be lost from yeast cells at rates up to 1% to 5% per generation. Stable maintenance of integrated sequences over generations obviates the addition of toxic antibiotics to the fermentation medium to exert selective pressure to maintain the sequences. The vectors of the invention also function well in yeast, by virtue of the yeast promoter sequence controlling the gene for antibiotic resistance. Furthermore, since industrial yeast strains are usually diploid or polyploid (as opposed to haploid laboratory strains), introduction into them of auxotrophic mutations (used for selection of transformants in haploid strains) is difficult. The use of antibiotic resistance in an integrating vector permits selection of stable transformants in any yeast strain, regardless of number of chromosomes or the presence or absence of specific mutations.

Introduction of genes encoding heterologous enzymes into industrial yeast strains using the vectors of the invention will facilitate the production of such products as alcohol, which ordinarily relies on sugars to feed the yeast. An enzyme such as glucoamylase will enable the yeast to break down starch from inexpensive sources such as tapioca and potatoes to yield glucose, which can be fed on by the yeast. Similarly, bread-making can be made cheaper when starch (flour) rather than sugar is used as the primary energy source.

Heterologous enzymes can also facilitate the production of light beer, which has a lower starch content than regular beer. One method currently used in light beer brewing to break down residual starch which remains after completion of the malting process is to add to the wort glucoamylase derived from bread mould, a step which can be eliminated where the yeast used in brewing also carries and expresses a glucoamylase encoding gene.

Other enzymes can facilitate commercial fermentation processes in other respects. For example, in wine-making, insertion of the gene for malolactic enzyme or malate permease will permit host yeast cells to convert metabolized malic acid from grapes, thus inhibiting spoilage of the wine by removing malic acid, which is otherwise fed on by spoilage bacteria.

Other features and advantages of the invention will be apparent from the following description of the preferred embodiments, and from the Claims.

We first briefly describe the drawings.

Figure 1 is a diagrammatic representation of a replicating vector.

Figures 2, 3a, and 3b are diagrammatic representations of integrating vectors of the invention.

Figure 4 is a diagrammatic representation of a mechanism by which an integrating vector is integrated into a host yeast chromosome.

### Plasmid Structure

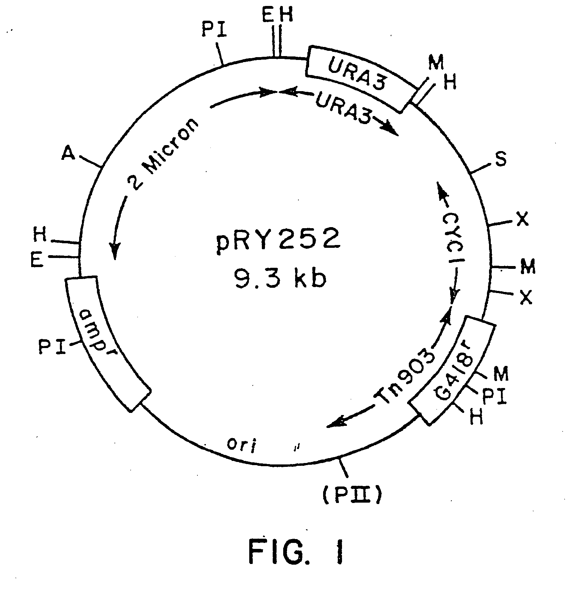

In Figs. 1-3, the following abbreviations are used for restriction endonuclease cleavage sites: A, XbaI; B, BamHI; E, EcoRI; H, HindIII; K, KpnI; M, SmaI; PI, PvuI; PII, PvuII; S, SaII; TII, SstII; U, StuI; X, XhoI. (A) denotes the position of a former XbaI site located about 3 kliobases away from the 5' end of the HO gene. This site was destroyed and replaced by a SaII site in the construction of pRY253. (PII) denotes a former PvuII site similarly destroyed during plasmid construction. Complete genes or gene fusions are shown by boxes. The abbreviations for the genes are as follows: amp^{r}, ampicillin resistance; G418^{r}, antibiotic G418 resistance; HO, homothallism; CYC1, iso-1-cytochrome c; URA3, orotidine-5'-monophosphate decarboxylase; GAL1, galactokinase; lacZ, beta-galactosidase. The concentric arrows inside the circles indicate segments of DNA whose origin is other than pBR322. The extent of these sequences is indicated by the arrowheads and the source is indicated by the labels. pBR322 sequences have no concentric arrows. Other abbreviations are: kb, kilobase pairs; ori, E. coli origin of replication.

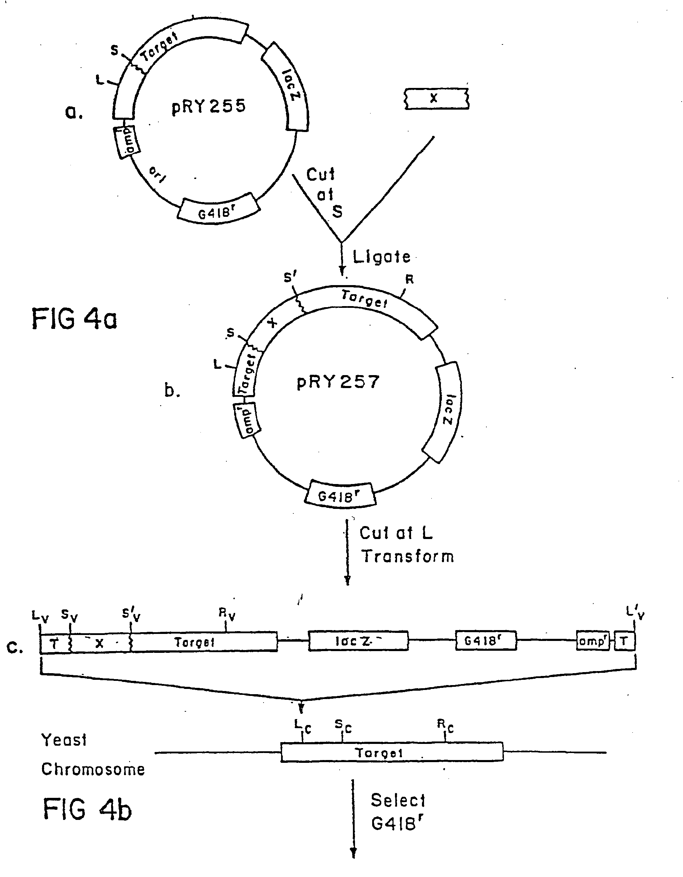

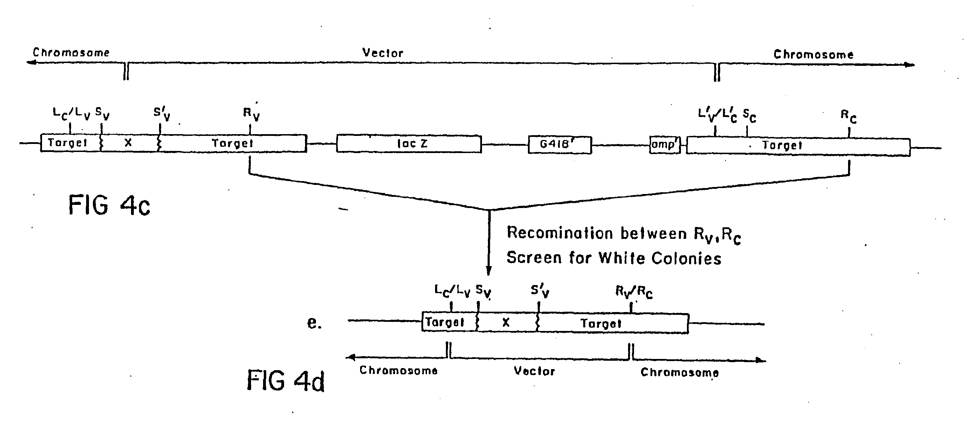

In Fig. 4, the following abbreviations are used: X, any gene or DNA sequence to be integrated into a yeast chromosome and expressed; S, a cloning site (for example: a restriction endonuclease site) into which gene X is inserted; L, a site (for example, a restriction endonuclease site) for linearizing the vector within the target sequence, "T" or "Target"; R, a site, not necessarily specific, where recombination between homologous vector-derived and chromosome-derived target sequences occurs. An apostrophe designates half of a site (such as S or L) that is separated from its other half by cleavage, by insertion of an intervening DNA sequence, or by integration into a chromosome. A subscript of V or C designates sites or portions of sites that are derived from the vector or chromosome, respectively. Other abbreviations are as in Fig. 1-3.

Referring to Fig. 1, replicating plasmid vector pRY252 is composed, beginning at the 12 o'clock position of the drawing and moving clockwise, of sequence E-H, a small piece of DNA from the E. coli plasmid pBR322; sequence H-H, which includes the yeast URA3 gene (one of the genes required for the ability to grow on uracil-deficient media; this gene is an unnecessary artifact in the plasmid which was originally inserted to provide a comparative selection means); sequence H-S, another piece of pBR322; sequence S-(PII), which includes the yeast CYCI (cytochrome c) promoter and most of the gene for resistance to G418 from the bacterial transposon Tn903 (the non-essential N-terminal region is not included); sequence (PII)-E, including the E. coli origin of replication from pBR322 and the amp^{r} gene for selecting transformants in E. coli; and sequence E-E, the yeast origin of replication from a yeast 2 micron circle.

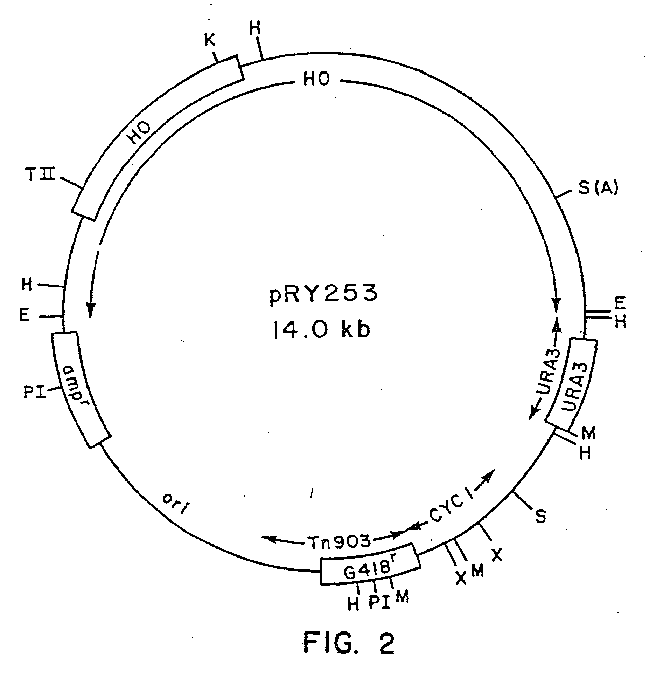

Referring to Figure 2, integrating plasmid vector pRY253 is derived from pRY252 in that the yeast origin of replication sequence is replaced by a 7.0 kb EcoRI fragment of S. cerevisiae containing the HO (homothallism) gene, including site K for insertion of a desired heterologous gene.

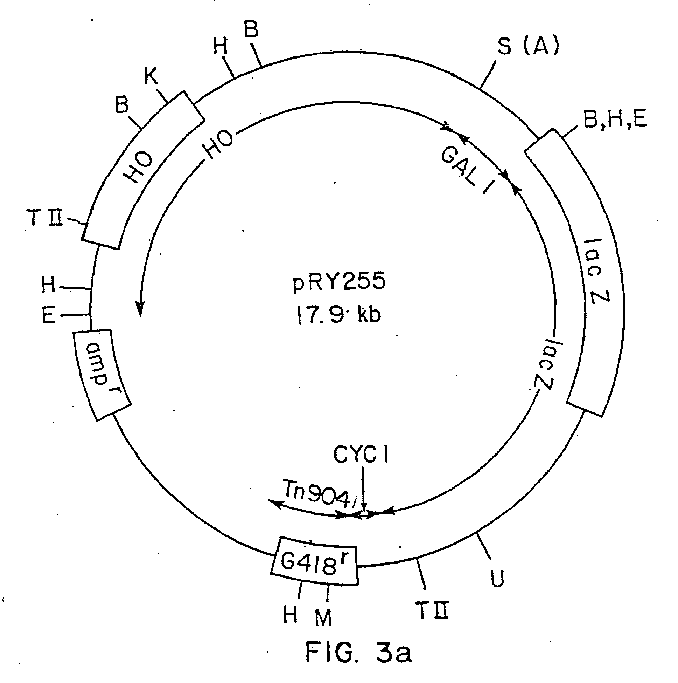

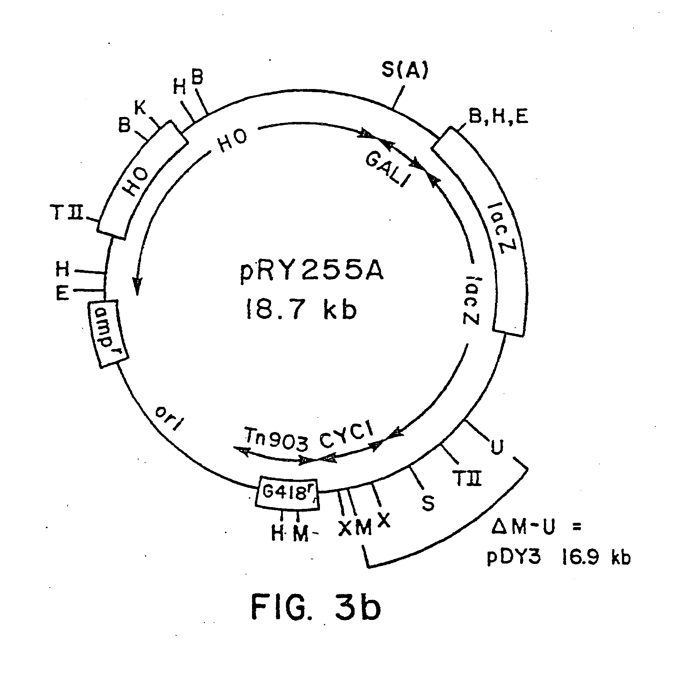

Referring to Fig. 3a, integrating plasmid vector pRY255 is derived from pRY253 in that a 3.3 kilobase SaII to XhoI fragment extending from one end of the HO insert to the beginning of the CYCl promoter sequence and containing the URA3 gene has been replaced with a 6.0 kilobase XhoI to Sall fragment containing a gene fusion of the yeast GAL1 gene and the E. coli lacZ gene. Referring to Fig. 3b, pRY255A is similar to pRY255, in that it is also derived from pRY253, in that the 6.0 kilobase XhoI to Sail fragment containing the GAL1-lacZ fusion is substituted for the 2.5 kilobase SaII fragment of pRY253.

Referring also to Figure 3b, pDY3 was derived from pRY255A by deleting the 1.8 kilobase region between a StuI site and, the SmaI site upstream from the CYC1 promoter.

pRY255A and pDY3 can be used in substantially the same way as pRY255, as described below. In addition, in pDY3, an Sstll site near the 3' end of the HO is unique on the vector, making it a more convenient site for linearization of the vector.

Plasmids pRY252, pRY253, pRY255, and pRY255A were deposited in the American Type Culture Collection, Rockville, Maryland, and have the depository numbers, respectively, ATCC 39687, 39688, 39689, and 39822.

Referring to Fig. 4, plasmid pRY257 will contain a gene X for a desired heterologous protein, e.g., glucoamylase or interferon, inserted at site S in plasmid pRY255.

### Plasmid Construction

The plasmids illustrated in Figs. 1-3 were made using conventional recombinant DMA methods and publicly available materials.

Plasmids pRY253, pRY255, pRY255A, and pDY3 were derived from replicating plasmid pRY252 which, briefly, was constructed as follows.

The URA3 gene was inserted into plasmid pBR322 as illustrated, and then the origin of replication from the endogenous yeast 2 micron circle, without the three genes normally accompanying it, was inserted. The vector is able to replicate in host yeast cells without containing these three genes, two of which encode proteins essential for replication, because host yeast cells already contain the endogenous 2 micron circle encoding those proteins (Botstein et al. (1979) Gene 8, 17).

The CYC1-G418^{r} fusion portion of the plasmid was constructed by fusing the XhoI site near the 5' end of - the G418^{r} gene of transposon Tn903 (described in Oka, et al. (1981) J. Mol. Biol. 147, 217) to the BamHI site following the CYC1 promoter and the 5' end of the CYC1 coding sequences of plasmid pLG669 (described in Guarente et al. (1981) PNAS USA 78, 2199) after rendering both ends flush with mung bean nuclease. The DNA sequence of this fusion junction is (CYCl) ... TAAATTAATAATGACCGGGCCG ... (G418^{r}).

Plasmids pRY253, pRY255, pRY255A, and pDY3 were constructed from pRY252 by making the gene fragment substitutions and deletions shown in the Figures. Plasmid pRY257 can be constructed by inserting a gene X for a desired protein at site S of pRY255, within the HO gene, so that there are portions of the HO gene on either side of gene X, as shown in Fig. 4.

### Plasmid Use.

The vectors of the invention can be used in any useful process in which host yeast cells express a desired heterologous gene. The desired heterologous gene can be inserted using conventional recombinant DNA techniques, e.g., as described in Maniatis et al. (1982) Molecular Cloning: A Laboratory Manual, Cold Spring Harber Press, Cold Spring Harbor, New York, hereby incorporated by reference. pRY253, pRY255, pRY255A, pDY3 can also be used to delete genes from wild type yeast strains. For the deletion of genes, the HO portion of the vectors becomes irrelevant. Deletion of a gene or a portion of a gene can be accomplished as follows:
1. Clone the gene to be deleted with some adjacent sequence on both sides of the gene.
2. Create a deletion of the cloned gene in vitro, leaving a portion of each of the 3' and 5' ends of the gene sufficient for homologous recombination but insufficient to encode the protein normally encoded by the gene.
3. Place the deletion-containing DNA at an appropriate location in one of the integration vectors described herein.
4. Linearize the vector at a point in one of the sequences adjacent to the deletion, and perform integration transformation, selecting for G418 resistance.
5. Grow a stable transformant for 20 to 40 generations non-selectively, and screen for vector jettisoning events by either loss of blue colony color on Xgal indicator plates or by replica plating to G418 containing plates.
6. Screen among colonies that have jettisoned the vector for those that retained the deleted version of the gene by Southern blotting.

The vectors are particularly useful in industrial yeast strains used in the production of end products such as wine, bread, and beer which involve carboydrate fermentation.

### Transformation

Yeast cells were transformed with vectors as follows.

Laboratory yeast strain DBY 745 (described in Guarente et al. (1981) PNAS USA 78, 2199); a Carlsberg® brewing strain (isolated from unpasteurized beer); Fleischman's® baking yeast (purchased at a supermarket); and a Bordeaux wine yeast (ATCC 42928) were grown in a standard rich medium, YEP-D, spheroplasted with glusulase, and exposed to plasmid DNA by standard methods of yeast transformation, as described in Sherman et al. (1981) Methods in Yeast Genetics (Cold Spring Harbor Laboratories Press, Cold Spring Harbor,. New York). Integrating plasmid pRY253 was linearized by restriction endonuclease digestion, at a unique SstII site near the 3' end of the HO gene, and pRY255 was linearized at a unique KpnI site near the 5' end-of the HO gene, priorto transformation, in order to direct integration at the HO locus. Replicating plasmid pRY252 was not linearized.

After exposure to the plasmids, 10⁸ spheroplasts were grown in YEP-D plus 1.0 M sorbitol for 30 minutes at 30°C and then plated in 6 ml of warm 3% agarose containing YEP-D over 20 ml of 2% agar. YEP-D, 1.0 M sorbitol, and 70 mM potassium phosphate, pH 7.0. After 10 minutes of cooling at room temperature, another 4 ml of warm 1% agar, YEP-D, 1.0 M sorbitol was layered over the top agar. The cells were then allowed to grow at 30°C for 6 generations, corresponding to 8 hours for DBY 745, 9 hours for Carlsberg, 7 hours for the wine yeast, and 6 hours for Fleishman's. After this "growing out" period, 0.6 ml of a sterile solution of antibiotic G418 at 25 mg/ml was spread over the agar surface and allowed to dry in a sterile hood. The plates were then incubated for 2-5 days at 30°C, after which time colonies appeared out of a background of untransformed cells. Several of these colonies were toothpicked onto fresh YEP-D plates containing 500 µg/ml G418. Cells that were successfully transformed gave rise to visible colonies within 24 hours, while untransformed cells did not. A summary of these results is given in Table 1, below.

**Table 1**

| Number of G418 resistant transformants per 10⁸ competent cells from 1 µg of plasmid DNA. | | | | |
|---|---|---|---|---|
| Strain | DNA | | | |
| | None | pRY252 | pRY253^{a} | pRY255^{b} |
| DBY 745 | 0 | 5,200 | 890 | 800 |
| Carlsberg | 0 | 260 | 13 | 7 |
| Wine Yeast (ATCC #42928) | 0 | 450 | 30 | 21 |
| Fleischman's | 0 | 510 | 22 | 17 |

| | | | | |
|---|---|---|---|---|
| ^{a} linearized with SstII prior to transformation. ^{b} linearized with KpnI prior to transformation | | | | |

Transformants obtained from the integrating plasmids pRY253 and pRY255 were shown to contain stably integrated plasmids, by growing isolated transformants for 10 to 20 generations non-selectively in YEP-D and showing that reversion to G418 sensitivity occurred at a rate less than 10⁻³. Transformants containing pRY255 gave blue colonies on plates containing galactose as the sole carbon source, 70 mM potassium phosphase buffer, pH 7.0, and Xgal indicator dye (5'-bromo-4'-chloro-3'-indoyl-beta-D-galactoside).

### Jettisoning of Vector Sequences

Plasmid vector pRY257 can be linearized and used to transform host yeast cells, as described above for plasmids pRY253 and pRY255. As described above, transformants are selected on the basis of antibiotic resistance (Fig. 4).

Following this selection, as shown in Fig. 4, a further step can be taken to jettison unnecessary portions of the vector which might adversely affect transformant stability, adversely affect the taste or any other important property of the end product, or waste metabolic energy. In effect, this screening step "deposits" the desired gene in the host chromosome, while excluding extraneous DNA. The exclusion of this extraneous DNA increases transformant stability by eliminating tandem repeat sequences which could cause undesirable recombination events resulting, for example, in loss of the desired heterologous gene. Also, elimination of the gene for antibiotic resistance can be an advantage if for some reason it is anticipated that the use of the antibiotic to kill the yeast may become necessary. Finally, elimination of all Escherichia coli derived sequences from the transformed yeast may simplify governmental regulatory clearance for use of the organism.

The screening depends on the presence in the vector of a gene encoding a screenable trait; in pRY257, this is the E. coli lacZ gene which encodes beta-galactosidase. Yeast colonies that express this gene tum blue on an appropriate indicator petri plate containing a colorimetric indicator dye such as XgaI. Thus to select transformants in which a portion of the vector DNA, including the lacZ gene, has been jettisoned, transformants are plated onto an indicator plate, and those colonies remaining white on the plates selected as the transformants, or descendants of transformants, not retaining the lacZ gene.

Fig. 4 illustrates the jettisoning mechanism. In some transformants there will be a cross-over event between vector sequences homologous with chromosome sequences (see Fig. 4d). This cross-over event causes the looping out and deletion of the region of the vector between the homologous sequences (see Fig. 4e). If desired heterologous gene X (encoding, say, glucoamylase) is outside this region, it remains deposited in the chromosome. The frequency of this type of event can be increased relative to that of other unwanted events (such as looping out of the entire plasmid including the deposited gene) by placing the deposited gene nearer to the end of the target sequences containing the linearization site than to the end of the target sequences that contain the "looping out" site.

The desired looping out event can be distinguished from undesired events by screening among white colonies for those that maintain gene X. This can be done either by a functional assay for the product of gene X (e.g., in the case of glucoamylase, halos on starch-containing plates), or by direct assay for the presence of gene X by Southern blotting techniques. The two screenings can be carried out at once, e.g. by using a medium containing both XgaI and starch.

Other embodiments are within the teaching of the invention.

For example, although the frequency at which the integrating vectors integrate into the host chromosome is increased by linearizing the vector prior to transformation, integration, at a lower frequency, can be achieved by transformation with the vectors in circularized form. Although the HO gene is the most preferred target gene, any other region of the host chromosome not involved in metabolism can be used. For example, the mutant homothallism gene of most laboratory yeast strains (the ho gene), which differs slightly from the wild-type HO gene, can be used as a target: the ho gene, like the HO gene, has the advantages of large size (about 2.000 base pairs) and non-involvement in metabolism in diploid or polyploid cells.

In addition to enzymes involved in the production of bread and alcoholic beverages, the vectors of the invention can be used in processes in which the desired end product is the protein, e.g., therapeutic proteins such as interferon, encoded by the inserted heterologous gene. The heterologous gene can also be a gene already carried on a different portion of the host chromosome; for example, it might be advantageous to add an additional copy of a native gene involved in alcohol production, to increase production levels.

The yeast promoter sequence controlling the gene for antibiotic resistance can also vary widely, the only crucial factor being that the sequence provides that a sufficient level of expression in yeast cells is maintained.

When a gene in the host chromosome is targeted by employing a vector containing a homologous sequence, linearization of the vector prior to transformation can occur anywhere within the homologous sequence; generally, however, integration efficiency is improved when linearization occurs near the center of the sequence, and decreases as the linearizaton point approaches either end of the sequence.

The screenable trait, in addition to the ability to produce beta-galactosidase, can be any trait whose absence can be detected. In addition, when beta-galactosidase production is used, the gene need not be the E. coli lacZ gene: for example, the LAC4 gene from Kluyeromyces species, e.g., K. lactis, which also encodes a beta-galactosidase, can also be used.

## Claims

1. A vector containing a DNA sequence capable of integration into a yeast chromosome comprising a yeast promoter sequence functionally coupled to a gene coding for resistance to antibiotic G418, such that the DNA sequence is capable of being directly selected for in the integrated state in a yeast cell transformed with said DNA.

2. A vector according to claim 1, in which the yeast promoter is a *Saccharomyces cerevisiae* promoter.

3. A vector according to claim 1 or claim 2, in which the promoter is a *Saccharomyces cerevisiae* iso-1-cytochrome C *(CYC1)* promoter.

4. A vector according to any preceding claim, in which said gene coding for resistance to antibiotic G418 is derived from transposon Tn903.

5. A vector according to any of the preceding claims, further comprising a gene encoding a desired heterologous protein.

6. A yeast cell transformed with a vector according to any of the preceding claims, or a descendent of such a yeast cell.

7. A method of transforming yeast cells comprising exposing a population of said yeast cells to a vector according to any of claims 1 to 5 under transforming conditions, exposing the cells to a dose of antibiotic G418 sufficient to prevent growth of untransformed cells of the population, and selecting cells that are capable of growing in the presence of said antibiotic.

8. A method for producing a desired heterologous protein comprising using a yeast cell prepared by a method according to claim 7 when dependent on claim 5 to produce said protein.

## Patentansprüche

1. Vektor, enthaltend eine DNA-Sequenz, die zur Integration in ein Hefechromosom in der Lage ist, umfassend eine Hefepromotorsequenz, die funktionell mit einem Gen gekoppelt ist, das die Resistenz gegen das Antibiotikum G418 kodiert, so dass die DNA-Sequenz in der Lage ist, direkt im integrierten Zustand in einer mit der DNA transformierten Hefezelle selektiert zu werden.

2. Vektor nach Anspruch 1, wobei der Hefepromotor ein Saccharomyces cerevisiae-Promotor ist.

3. Vektor nach Anspruch 1 oder Anspruch 2, wobei der Promotor ein Saccharomyces cerevisiae-Iso-1-Cytochrom C- (CYC1)-Promotor ist.

4. Vektor nach einem der vorangegangenen Ansprüche, wobei sich das Gen, das die Resistenz gegen das Antibiotikum G418 kodiert, vom Transposon Tn903 ableitet.

5. Vektor nach einem der vorangegangenen Ansprüche, der ferner ein Gen umfasst, das ein gewünschtes heterologes Protein kodiert.

6. Hefezelle, die mit einem Vektor nach einem der vorangegangenen Ansprüche transformiert ist, oder ein Deszendent einer solchen Hefezelle.

7. Verfahren zum Transformieren von Hefezellen, umfassend das Aussetzen einer Population von Hefezellen einem Vektor nach einem der Ansprüche 1 bis 5 unter Transformierbedingungen, Aussetzen der Zellen einer Dosis des Antibiotikums G418, die ausreicht, das Wachstum von nichttransformierten Zellen der Population zu verhindern, und Selektieren der Zellen, die in der Lage sind, in Gegenwart des Antibiotikums zu wachsen.

8. Verfahren zur Herstellung eines gewünschten heterotogen Proteins, umfassend die Verwendung einer Hefezelle, die durch ein Verfahren nach Anspruch 7 produziert wurde, wenn von Anspruch 5 abhängig, um das Protein zu produzieren.

## Revendications

1. Vecteur contenant une séquence d'ADN capable d'intégration dans un chromosome de levure comprenant une séquence promotrice de levure fonctionnellement couplée à un gène codant pour la résistance à l'antibiotique G418, de telle sorte que la séquence d'ADN peut être directement choisie à l'état intégré dans une cellule de levure transformée avec ledit ADN.

2. Vecteur selon la revendication 1, dans lequel le promoteur de la levure est un promoteur de *Saccharomyces cerevisiae.*

3. Vecteur selon la revendication 1 ou la revendication 2, dans lequel le promoteur est un promoteur iso-1-cytochrome C *(CYC1)* de *Saccharomyces cerevisiae.*

4. Vecteur selon l'une quelconque des revendications précédentes, dans lequel ledit gène codant pour la résistance à l'antibiotique G418 est dérivé du transposon Tn903.

5. Vecteur selon l'une quelconque des revendications précédentes, comprenant en outre un gène codant pour une protéine hétérologue souhaitée.

6. Cellule de levure transformée avec un vecteur selon l'une quelconque des revendications précédentes, ou un descendant d'une telle cellule de levure.

7. Procédé de transformation de cellules de levure comprenant l'exposition d'une population desdites cellules de levure à un vecteur selon l'une quelconque des revendications 1 à 5 dans des conditions de transformation, l'exposition des cellules à une dose d'antibiotique G418 suffisante pour empêcher la croissance des cellules non transformées de la population, et la sélection de cellules qui sont capables de se développer en présence dudit antibiotique.

8. Procédé de production d'une protéine hétérologue souhaitée comprenant l'utilisation d'une cellule de levure préparée par un procédé selon la revendication 7 lorsqu'elle dépend de la revendication 5 pour produire ladite protéine.
